Europäisches Patentamt

European Patent Office

Office européen des brevets

(11) Publication number: **0 352 052**
**A2**

# EUROPEAN PATENT APPLICATION

(21) Application number: **89307241.3**

(51) Int. Cl.5: **A 01 N 63/02**

(22) Date of filing: **17.07.89**

(30) Priority: **18.07.88 US 220778   19.06.89 US 366123**

(43) Date of publication of application:
**24.01.90 Bulletin 90/04**

(84) Designated Contracting States:
**AT BE CH DE ES FR GB GR IT LI LU NL SE**

(71) Applicant: **Mycogen Corporation**
**5451 Oberlin Drive**
**San Diego, CA 92121 (US)**

(72) Inventor: **Schwab, George E.**
**316 Prospect Avenue**
**La Jolla California 92037 (US)**

**Halloran, Timothy P.**
**3694 Via Baldona**
**Oceanside California 92056 (US)**

(74) Representative: **Perry, Robert Edward et al**
**GILL JENNINGS & EVERY 53-64 Chancery Lane**
**London WC2A 1HN (GB)**

(54) Use of a gene product of a bacillus thuringiensis var. israelensis.

(57) The invention concerns the use of a gene product from a Bacillus thuringiensis var. israelensis (B.t.i.) which is active against adult nematode worms and larvae. This B.t.i. gene product can be used to treat animals and plants hosting susceptible nematodes.

EP 0 352 052 A2

## Description

## USE OF A GENE PRODUCT OF A BACILLUS THURINGIENSIS VAR. ISRAELENSIS

### Field of the Invention

This invention relates to the use of a gene product of a Bacillus thuringiensis var. israelensis.

### Background of the Invention

The bacterium Bacillus thuringiensis is widely used for microbial control of insects. The bacterium produces a proteinaceous paraspore or crystal designated as delta endotoxin which is toxic to a variety of insects. Following ingestion the endotoxin is processed by gut proteases from the protoxin form to the active toxin form. The active toxin disrupts the gut epithelial cell membrane resulting in an ionic imbalance and cytolysis (Knowles, B.H. et al. [1984] Febs 168:197-202; McCarthy, W. et al. [1988] In Vitro Cell. Develop. Biol. 24:59-64).

The activity of Bacillus thuringiensis delta endotoxin includes the insect order Diptera. Specifically, Bacillus thuringiensis var. israelensis is lethal for mosquito larvae including genera Aedes and Culex (Goldberg, L.J. and Margalitt, J. [1977] Mosquito news 37:355-358). Bacillus thuringiensis var. israelensis contains three major inclusion types which have been shown to exhibit synergism in mosquitocidal activity (Ibarra, J.E. and Federici, B.A. [1986] J. Bacteriology 165:527-533; Wu, D. and Chang, F.N. [1985] Febs 190:232-236).

In addition to mosquitocidal activity, crystal enriched preparations of Bacillus thuringiensis var. israelensis have been shown to be ovicidal and larvacidal for the ruminant parasitic nematode Trichostrongylus colubriformis (Bottjer, K.P. and Bone, L.W. [1987] J. Nematology 19:282-286). However, it is of importance to note that the analogy between dipteran and nematicidal activity has not been made. In fact, Bone, L.W. et al (1985) Exp. Parasitol., 60:314-322, report that the delta-endotoxin of Bacillus thuringiensis var. israelensis was not responsible for nematicidal activity. Furthermore, in a study of Bottjer, K.P. et al, (1985) Exp. Parasitol., 60:239-244, the nematicidal activity of 30 strains of Bacillus thuringiensis were tested with the result that Bacillus thuringiensis var. israelensis was placed 14th.

At the present time, there is a need to have more effective means to control the many nematodes that cause considerable damage to susceptible hosts.

EP-A-0308199 discloses a gene product derived from a Bacillus thuringiensis, that is shown to have activity only against dipteran insects, e.g. mosquitos. The gene was derived from a B. thuringiensis var. israelensis (B.t.i.) microbe identified as strain HD567.

### Summary of the Invention

According to this invention, the gene product as described in EP-A-0308199 is used to control nematodes.

More specifically, the subject invention concerns the use of DNA encoding an ca. 130 kilodalton (kd) protein having activity against nematodes. Formulations and methods for use of the B.t.i. toxin to control nematodes are disclosed.

### Detailed Disclosure of the Invention

The B.t.i. isolate used as the source of the toxin gene of the subject invention is identified as B.t.i. strain HD567. This culture is available from Howard T. Dulmage, Cotton Insects Laboratory, United States Department of Agriculture Laboratory, P.O. Box 1033, Brownsville, TX 78520. This culture can be grown using standard techniques and media well known in the art. The B.t.i. cells can be harvested by standard techniques, e.g., centrifugation. The recovered B.t.i. cells are then converted to protoplasts by standard methods. Thereafter cellular DNA is extracted by well-known procedures. The resulting purified DNA is the starting material for digestion with suitable restriction endonucleases.

A gene bank of B.t.i. can then be constructed. In the subject invention, the purified B.t.i. DNA, obtained as described above, was digested with the restriction endonucleases EcoRI and HindIII. The EcoRI and HindIII fragment, after purification, was ligated into the well-known and available plasmid pUC19 which had been cleaved with EcoRI and HindIII.

Once the gene bank of B.t.i. DNA was constructed, it then became necessary to construct a DNA probe to screen the gene bank. The probe was labelled and hybridized by procedures known in the art. The net result was the detection of positive clones, i.e., those aht hybridized to the constructed probe.

The recombinant plasmids isolated from representative positive clones were found to have an ca. 3.4 kilobase (kb) EcoRI-HindIII DNA fragment. This ca. 3.4 kb DNA fragment was sequenced by the standard Sanger dideoxy chain termination method.

In order to facilitate efficient expression of the cloned B.t.i. gene in E. coli, it was necessary to insert a synthetic ologonucleotide at the EcoRI site to restore the ATG start codon and to introduce a Shine-Dalgarno region.

The cellular host containing the B.t.i. nematicidal gene may be grown in any convenient nutrient medium, where the DNA construct provides a selective advantage, providing for a selective medium so that substantially all or all of the cells retain the B.t.i. gene. These cells may then be harvested in accordance with conventional ways. Alternatively, the cells can be treated prior to harvesting.

The novel gene product of the invention shows activity against a tested nematode. The group of diseases described generally as helminthiasis is due to infection of an animal host with parasitic worms known as helminths. Helminthiasis is a prevalent and serious economic problem in domesticated animals such as swine, sheep, horses, cattle, goats, dogs, cats and poultry. Among the helminths, the group of worms described as nematodes causes widespread and often times serious infection in various species of animals. The most common genera of nematodes infecting the animals referred to above are Haemonchus, Trichostrongylus, Ostertagia, Nematodirus, Cooperia, Ascaria, Bunostromum, Oesophagostomum, Chabertia, Trichuris, Strongylus, Trichonema, Dictyocaulus, Capillaria, Heterakis, Toxocara, Ascaridia, Oxyuris, Ancylostoma, Uncinaria, Toxascaris, Caenorhabditis, and Parascaris. Certain of these, such as Nematodirus, Cooperia, and Oesophagostomum, attack primarily the intestinal tract, while others, such as Dictyocaulus are found in the lungs. Still other parasites may be located in other tissues and organs of the body.

The toxin of the invention is useful as a nematocide for the control of soil nematodes and plant parasites selected from the genera Bursaphalenchus, Criconemella, Ditylenchus, Globedera, Helicotyienchus, Heterodera, Melodiogyne, Pratylenchus, Radolpholus, Rotelynchus, or Tylenchus.

Alternatively, because some plant parasitic nematodes are obligate parasites, genes coding for nematocidal B.t. toxins can be engineered into plant cells to yield nematode-resistant plants. The methodology for engineering plant cells is well established (cf. Nester, E.W., Gordon, M.P., Amasino, R.M. and Yanofsky, M.F. [1984] Ann. Rev. Plant Physiol. 35:387-399).

The B.t.i. toxin of the invention can be administered orally in a unit dosage form such as a capsule, bolus or tablet, or as a liquid drench when used as an anthelmintic in mammals. The drench is normally a solution, suspension or dispersion of the active ingredient, usually in water, together with a suspending agents such as bentonite and a wetting agent or like excipient. Generally, the drenches also contain an antifoaming agent. Drench formulations generally contain from about 0.001 to 0.5% by weight of the active compound. Preferred drench formulations may contain from 0.01 to 0.1% by weight. The capsules and boluses comprise the active ingredient admixed with a carrier vehicle such as starch, talc, magnesium stearate, or dicalcium phosphate.

Where it is desired to administer the toxin compound in a dry, solid unit dosage form, capsules, boluses or tablets containing the desired amount of active compound usually are employed. These dosage forms are prepared by intimately and uniformly mixing the active ingredient with suitable finely divided diluents, fillers, disintegrating agents and/or binders such as starch, lactose, talc, magnesium stearate, vegetable gums and the like. Such unit dosage formulations may be varied widely with respect to their total weight and content of the antiparasitic agent, depending upon factors such as the type of host animal to be treated, the severity and type of infection and the weight of the host.

When the active compound is to be administered via an animal feedstuff, it is intimately dispersed in the feed or used as a top dressing or in the form of pellets which may then be added to the finished feed or, optionally, fed separately. Alternatively, the antiparasitic compound may be administered to animals parenterally, for example, by intraruminal, intramuscular, intratracheal, or subcutaneous injection, in which event the active ingredient is dissolved or dispersed in a liquid carrier vehicle. For parenteral administration, the active material is suitably admixed with an acceptable vehicle, preferably of the vegetable oil variety, such as peanut oil, cottonseed oil and the like. Other parenteral vehicles, such as organic preparations using solketal, glycerol, formal and aqueous parenteral formulations, are also used. The active compound is dissolved or suspended in the parenteral formulation for administration; such formulations generally contain from 0.005 to 5% by weight of the active compound.

When the toxin is administered as a component of the feed of the animals, or dissolved or suspended in the drinking water, compositions are provided in which the active compound or compounds are intimately dispersed in an inert carrier or diluent. By inert carrier is meant one that will not react with the antiparasitic agent and one that may be administered safely to animals. Preferably, a carrier for feed administration is one that is, or may be, an ingredient of the animal ration.

Suitable compositions include feed premixes or supplements in which the active ingredient is present in relatively large amounts and which are suitable for direct feeding to the animal or for addition to the feed either directly or after an intermediate dilution or blending step. Typical carriers or diluents suitable for such compositions include, for example, distillers' dried grains, corn meal, citrus meal, fermentation residues, ground oyster shells, wheat shorts, molasses solubles, corn cob meal, edible bean mill feed, soya grits, crushed limestone and the like.

The gene product of the subject invention can be introduced into microbes capable of occupying, surviving in and proliferating in the phytosphere of plants according to the procedures disclosed in European Patent Applicaton 0 200 344. Upon ingestion of such a plant by an animal hosting a nematode, the nematode-active toxin becomes available in the animal host to control the nematode infestation.

A wide variety of ways are available for introducing the B.t.i. gene expressing the toxin into the microorganism host under conditions which allow for stable maintenance and expression of the gene. One can provide for DNA constructs which include the transcriptional and translational regulatory signals for expression of the toxin gene, the toxin gene under their regulatory control and a DNA sequence homologous with a sequence in the host organism, whereby integration will occur, and/or a replication system which is functional in the host, whereby integration or stable maintenance will occur.

The transcriptional initiation signals will include a promoter and a transcriptional initiation start site. In some instances, it may be desirable to provide for regulative expression of the toxin, where expression of the toxin will only occur after release into the environment. This can be achieved with operators or a region binding to an activator or enhancers, which are capable of induction upon a change in the physical or chemical environment of the microorganisms. For example, a temperature sensitive regulatory region may be employed, where the organisms may be grown up in the laboratory without expression of a toxin, but upon release into the environment, expression would begin. Other techniques may employ a specific nutrient medium in the laboratory, which inhibits the expression of the toxin, where the nutrient medium in the environment would allow for expression of the toxin. For translational initiation, a ribosomal binding site and an initiation codon will be present.

Various manipulations may be employed for enhancing the expression of the messenger, particularly by using an active promoter, as well as by employing sequences, which enhance the stability of the messenger RNA. The initiation and translational termination region will involve stop codon(s), a terminator region, and optionally, a polyadenylation signal.

In the direction of transcription, namely in the 5′ to 3′ direction of the coding or sense sequence, the construct will involve the transcriptional regulatory region, if any, and the promoter, where the regulatory region may be either 5′ or 3′ of the promoter, the ribosomal binding site, the initiation codon, the structural gene having an open reading frame in phase with the initiation codon, the stop codon(s), the polyadenylation signal sequence, if any, and the terminator region. This sequence as a double strand may be used by itself for transformation of a microorganism host, but will usually be included with a DNA sequence involving a marker, where the second DNA sequence may be joined to the toxin expression construct during introduction of the DNA into the host.

By a marker is intended a structural gene which provides for selection of those hosts which have been modified or transformed. The marker will normally provide for selective advantage, for example, providing for biocide resistance, e.g., resistance to antibiotics or heavy metals; complementation, so as to provide prototrophy to an auxotrophic host, or the like. Preferably, complementation is employed, so that the modified host may not only be selected, but may also be competitive in the field. One or more markers may be employed in the development of the constructs, as well as for modifying the host. The organisms may be further modified by providing for a competitive advantage against other wild-type microorganisms in the field. For example, genes expressing metal chelating agents, e.g., siderophores, may be introduced into the host along with the structural gene expressing the toxin. In this manner, the enhanced expression of a siderophore may provide for a competitive advantage for the toxin-producing host, so that it may effectively compete with the wild-type microorganisms and stably occupy a niche in the environment.

Where no functional replication system is present, the construct will also include a sequence of at least 50 basepairs (bp), preferably at least about 100 bp, and usually not more than about 1000 bp of a sequence homologous with a sequence in the host. In this way, the probability of legitimate recombination is enhanced, so that the gene will be integrated into the host and stably maintained by the host. Desirably, the toxin gene will be in close proximity to the gene providing for complementation as well as the gene providing for the competitive advantage. Therefore, in the event that a toxin gene is lost, the resulting organism will be likely to also lose the complementing gene and/or the gene providing for the competitive advantage, so that it will be unable to compete in the environment with the gene retaining the intact construct.

A large number of transcriptional regulatory regions are available from a wide variety of microorganism hosts, such as bacteria, bacteriophage, cyanobacteria, algae, fungi, and the like. Various transcriptional regulatory regions include the regions associated with the trp gene, lac gene, gal gene, the lambda left and right promoters, the Tac promoter, the naturally-occurring promoters associated with the toxin gene, where functional in the host. See for example, U.S. Patent Nos. 4,332,898, 4,342,832 and 4,356,270. The termination region may be the termination region normally associated with the transcriptional initiation region or a different transcriptional initiation region, so long as the two regions are compatible and functional in the host.

Where stable episomal maintenance or integration is desired, a plasmid will be employed which has a replication system which is functional in the host. The replication system may be derived from the chromosome, an episomal element normally present in the host or a different host, or a replication system from a virus which is stable in the host. A large number of plasmids are available, such as pBR322, pACYC184, RSF1010, pRO1614, and the like. See for example, Olson et al., (1982) J. Bacteriol. 150:6069, and Bagdasarian et al., (1981) Gene 16:237, and U.S. Patent Nos. 4,356,270, 4,362,817, and 4,371,625.

The B.t.i. gene can be introduced between the transcriptional and translational initiation region and the transcriptional and translational termination region, so as to be under the regulatory control of the initiation region. This construct will be included in a plasmid, which will include at least one replication system, but may include more than one, where one replication system is employed for cloning during the development of the plasmid and the second replication system is necessary for functioning in the ultimate host. In addition, one or more markers may be present, which have been described previously. Where integration is desired, the plasmid will desirably include a sequence homologous with the host genome.

The transformants can be isolated in conventional manner, usually employing a selection technique, which allows for selection of the desired organism as against unmodified organisms or transferring organisms, when present. The transformants then can be tested for pesticidal activity.

The preparation of a plasmid identified as pBTI3,82-5, containing the B.t.i. toxin gene, is described in EP-A-0308199.

A subculture of E. coli BB3 (pBTl3,82-5) has been deposited as described in EP-A-0308199. The accession number is NRRL B-18252.

The nucleotide sequence encoding the B.t.i. toxin gene (1143 bases including the "end" signal), and the deduced amino-acid sequence, are shown in claim 1 of EP-A-0308199.

It is well known in the art that the amino-acid sequence of a protein is determined by the nucleotide sequence of the DNA. Because of the redundancy of the genetic code, i.e. that more than one coding nucleotide triplet (codon) can be used for most of the amino-acids use to make proteins, different nucleotide sequences can code for a particular amino-acid.

Thus, the amino-acid sequence of B.t.i. toxin can be prepared by equivalent nucleotide sequences encoding the same amino-acid sequence of the protein. Accordingly, the subject invention includes the use of such equivalent nucleotide sequences. In addition, it has been shown that proteins of identified structure and function may be constructed by changing the amino-acid sequence if such changes do not alter the protein secondary structure (Kaiser, E.T. and Kezdy, F.J. (1984) Science 223:249-255). Thus, the subject invention includes the use of mutants of the amino-acid sequence depicted in EP-A-0308199 which do not alter the protein secondary structure, or if the structure is altered, the nematicidal activity is retained to some degree.

The following Example illustrates the invention.

### Example

The ca. 3.4 kb EcoR1-HindIII fragment containing the coding region of the 130 kd B.t.i. toxin gene from HD567 (see EP-A-0308199) was cloned into the EcoR1-HindIII sites of the tac promoter-containing expression vector, pkk223-3 (Pharmacia). A 32-mer synthetic oligonucleotide (A) was inserted at the EcoR1 site to restore the ATG start codon and to introduce a Shine-Dalgarno region. The resulting plasmid, pMYC-383, was introduced into E. coli JM101 to give E. coli MR383. The expression of B.t.i. toxin by this plasmid/host combination is verified by its identification on a polyacrylamide-SDS gel.

(A) GAATTCTGCA GTAAGGAGGT GTATATAATG AA

The synthetic oligonucleotide introduced at the EcoR1 site is shown with the Shine-Dalgarno region underlined and the initiation codon in boldface.

C. elegans was cultured as described by Simpkin and Coles (J. Chem. Tech. Biotechnol. 31:66-69, 1981) in Corning (Corning Glass Works, Corning, NY) 24-well tissue culture plates containing 1 ml S-basal media, 0.05 mg ampicillin and 0.01 mg cholesterol. Each well also contained ca. $10^8$ cells of E. coli strain OP-50 (a uracil auxotroph). The wells were seeded with ca. 100-200 nematodes each and incubated at 20°C. 100μl samples of E. coli strain JM 101 containing the gene expression product of the plasmid pMYC-383 were added to the wells by serial dilution at a final concentration range spanning $10^8$-$10^3$ cells per well. 100 μl samples of the expression vector without the B.t.i. gene were added at the same range to the other wells to serve as controls. All experiments were performed in duplicate and repeated 2 times.

Each of the wells were examined daily, and the results are as follows:

#### 24 hours

| No. E. coli per wells | Activity | |
|---|---|---|
| | Control | pMYC-383 |
| $10^8$ | All worms active | No live worms |
| $10^7$ | All worms active | No live worms |
| $10^6$ | All worms active | 50% live worms |
| $10^5$ | All worms active | All worms active |
| $10^4$ | All worms active | All worms active |
| $10^3$ | All worms active | All worms active |

Repeat Experiment:

#### 24 hours

| No.E. coli per wells | Activity | |
|---|---|---|
| | Control | pMYC-383 |
| $10^8$ | All worms active | < 1% live worms |
| $10^7$ | All worms active | < 1% live worms |
| $10^6$ | All worms active | < 1% live worms |
| $10^5$ | All worms active | 50% live worms |
| $10^4$ | All worms active | All worms active |
| $10^3$ | All worms active | All worms active |

Following 144 hours, neonates began to hatch in both the control and treatment wells and there was no apparent significant difference between the two groups in terms of mortality.

### Claims

1. A process for controlling nematodes, which comprises contacting the nematodes with a toxin produced by a Bacillus thuringiensis gene product of clone pMYC-383.

2. A process according to claim 1, wherein the toxin is active against nematodes selected from the genera Bursaphalenchus, Criconemella, Ditylenchus, Globedera, Helicotylenchus, Heterodera, Melodiogyne, Pratylenchus, Radolpholus, Rotelynchus, and Tylenchus.

3. A process according to claim 1, wherein the nematode is selected from the genera Haemonchus, Trichostrongylus, Ostertagia, Nematodirus, Cooperia, Ascaris, Bunostomum, Oesophagostomum, Chabertia, Trichuris, Strongylus, Trichonema, Dictylocaulus, Capillaria, Heterakis, Toxocara, Ascaridia, Oxyuris, Ancylostoma, Uncinaria, Toxascaris, Caenorhabditis, and Parascaris.

4. A process according to claim 3, wherein the nematode is Caenorhabditis elegans or Haemonchus contortus.

5. A process according to any preceding claim, wherein the toxin gene is genetically engineered into a plant or a different mircobe capable of occupying, surviving in, and proliferating in the phytosphere of plants.

6. A plant containing a Bacillus thuringiensis gene product obtained from a Bacillus thuringiensis gene product of clone pMYC-383.

7. Use of a Bacillus thuringiensis gene product of clone PMYC-383 for the manufacture of a medicament for use in the treatment of an animal hosting a nematode.

8. Use according to claim 7, wherein the nematode is as defined in claim 3 or claim 4.

9. Use according to claim 7 or claim 8, wherein the toxin gene is as defined in claim 5.